# EUROPEAN PATENT APPLICATION

(11) **EP 2 660 267 A1**
(43) Date of publication of application: **06.11.2013**
(21) Application number: 12382166.2
(22) Date of filing: 04.05.2012
(51) Int. Cl.: C08G 65/00, A61K 49/04, C07C 25/00

(54) **Degradable polymeric contrast agents for cancer diagnostic**

(71) Applicant: Nanomolecular (Asociacion de Interes Economico), 08034 Barcelona (ES)
(72) Inventor: Peña Gulín, Oscar, 08027 Barcelona (ES)
(74) Representative: ABG Patentes, S.L.

(57) **Abstract**

The present invention relates to polyacetal polymers which comprise repeating structural units of formula (I), being each of these repeating structural units equal or different 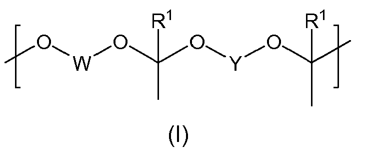 wherein at least one of the repeating structural units of formula (I) contains a group W selected from a triiodobenzene derivative biradical of formula (II) and of formula (III)

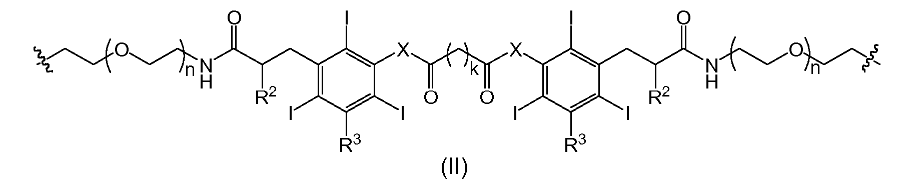

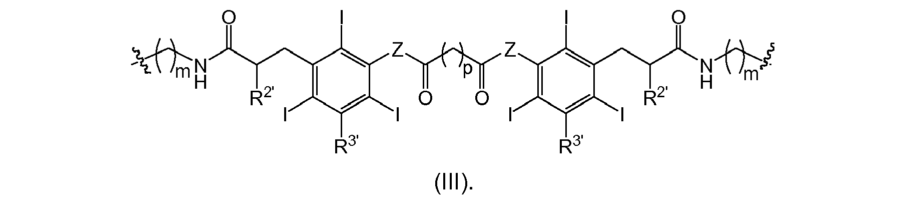

The invention also refers to a process for their preparation, to compositions comprising them, and to their use as contrast agents, especially for cancer diagnostic.

## Description

### Field of the Invention

The present invention relates to polymers comprising triiodobenzene derivatives, to a process for their preparation and to compositions comprising them, as well as to their use as contrast agent, preferably for cancer diagnostic.

### Background of the Invention

Polyiodinated benzene derivatives are well known as contrast agents due to their capacity to efficiently absorb X-rays. These iodinated compounds have been ordinarily used over the years for urographies and angiographies. Several polyiodinated benzene derivatives are widely used for these applications, but 1,3,5-triodobenzene derivatives are the most common in the market (loversol, lohexol, lopamidol, ...).

Due to the small size of iodinated contrast agents, they diffuse rapidly once injected intravenously and therefore imaging must be done rapidly after their administration in order to provide useful contrast.

Triiodobenzene-polymer conjugates have been already used for X-ray imaging in different applications. EP0809519 discloses the use of triiodobenzene-polymer conjugates in oral or rectal formulations for the imaging of gastrointestinal tract by X-ray contrast. Also, US5746998 discloses the use of block-copolymers with conjugated triiodobenzene derivatives to form micelles, which are useful in X-ray imaging.

In view of the prior art, triiodobenzene-polymers that are stable in the bloodstream and provide good targeting and accumulation at the desired tissue are still needed, especially as contrast agents for cancer diagnosis. Biocompatible and degradable polymers that are easily degraded when they reach the target tissue, would be desirable. Though some polymeric contrast agents have been disclosed, they have not been introduced in the market.

### Summary of the Invention

The present invention relates to novel polymers comprising triiodobenzene groups of formulae (VI) and (VII) as part of their backbone for their use in X-ray imaging. In particular, they have been found to be especially efficient in cancer diagnosis.

The inventors have found that biocompatible polymers having a molecular weight from 5,000 to 500,000 g/mol, which comprise a polyacetal backbone of randomly repeating structural units of formula (I), being these repeating structural units equal or different, wherein at least one of these repeating units contains a triiodobenzene compound, are useful for efficient cancer diagnosis by X-ray imaging. These hydrophobic triiodobenzene monomeric groups change the hydrodynamic properties of the polymers in physiological aqueous conditions, making them more suitable for cancer diagnosis.

The inventors have observed that the polymers of the invention accumulate in tumor tissues, thus providing a high contrast in these tissues without the need to attach specific targeting ligands. It has been found out that, due to the presence of the hydrophobic triiodobenzene monomeric groups, these polymers aggregate under physiological conditions forming particles large enough to be selectively accumulated in tumors, and not in sane tissues. Particle sizes higher than the renal threshold (similar to the size of a medium-large protein such as HSA, human serum albumin, Rh = 3.6 nm) are observed thus also avoiding renal elimination.

Consequently, said compounds can be used in the diagnosis of cancer. Triiodobenzene derivatives are radiopaque contrast agents that efficiently absorb X-rays and, thus, the polymers of the invention can be used in X-ray imaging.

These polymers are biodegradable which ensures their further elimination from the body.

Compared to the state of the art, the polymers of the invention provide better targeting and accumulation of triiodobenzene in tumor tissues improving the contrast, for example by X-ray imaging, compared to non-polymeric triiodobenzene derivatives used commonly for diagnosis.

Besides, compared to other triodobenzene-polymers described in the state of the art, the polymers of the present invention show a pH-dependent degradation rate. The environment of tumor tissues is slightly acid (pH 6.0-7.0) in comparison to the blood stream (pH 7.3-7.4). In some particular cases, such as breast cancer, the environment of the tumor tissues is particularly more acid (pH 5.7-6.7). The polymers of the present invention are stable in the bloodstream and ensure their biocompatibility by their pH-dependent degradability at the target site (the tumor tissue). It has been also observed that the triiodobenzene monomers released when the polymers are degraded at the tumor tissue provide a better contrast since they do not rapidly diffuse.

Furthermore, the polymers of the present invention are non-toxic and biocompatible.

Thus, in a first aspect, the invention is directed to a polymer having a molecular weight from 5,000 to 500,000 g/mol which comprises repeating structural units of formula (I), being each of these repeating structural units equal or different 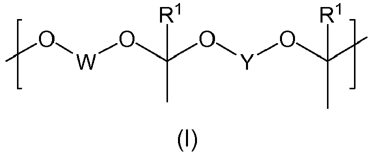 and wherein at least one of the repeating structural units of formula (I) contains a group W selected from a biradical of formula (II) and a biradical of formula (III)

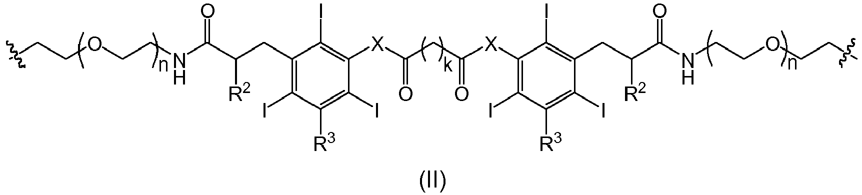

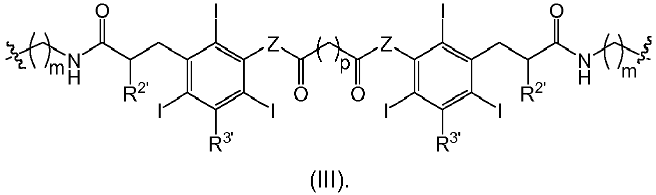

In another aspect, the invention is directed to triiodobenzene compounds of formulae (VI) and (VII). These compounds are useful intermediates in the preparation of the polymers of the invention.

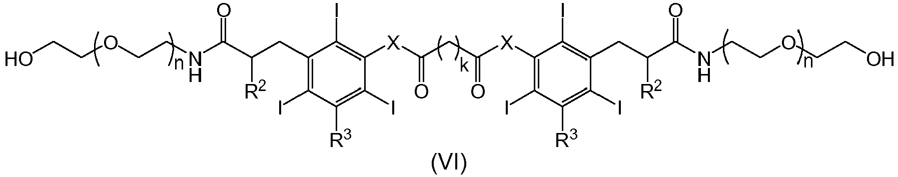

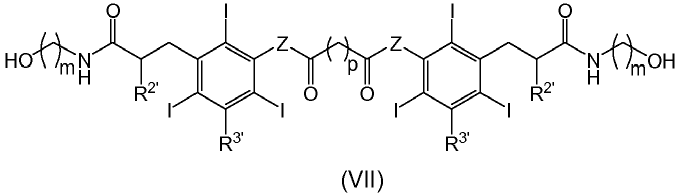

Another aspect refers to a process for preparing the polymers of the invention.

In another aspect, the invention refers to a composition comprising a polymer of the invention and a pharmaceutically acceptable carrier.

In another aspect, the invention is directed to a polymer of the invention, for use in medicine.

In a further aspect, the invention refers to a polymer of the invention, for use as a contrast agent.

### Brief Description of the Figures

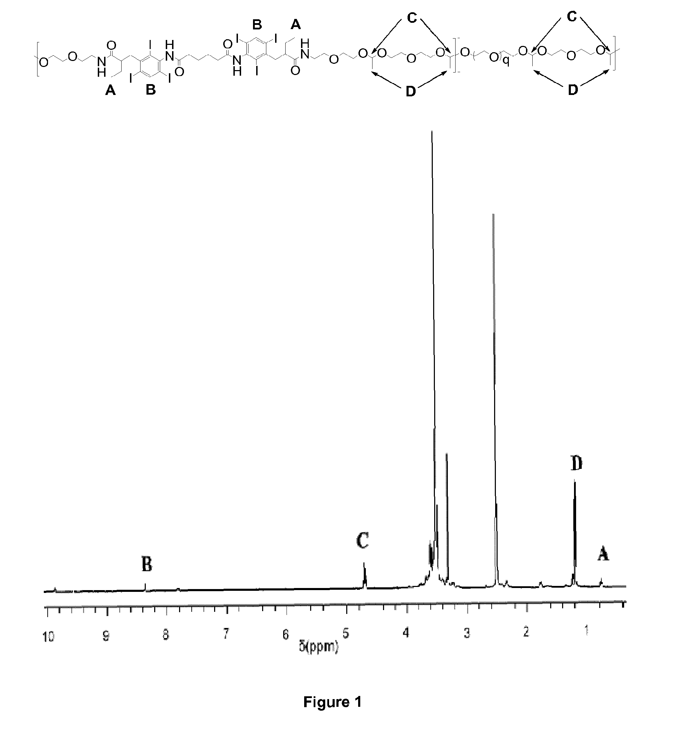

Figure 1. ¹H-NMR spectrum (DMSO-d₆, 400 MHz) of a polymer of the invention (polymer **4**).

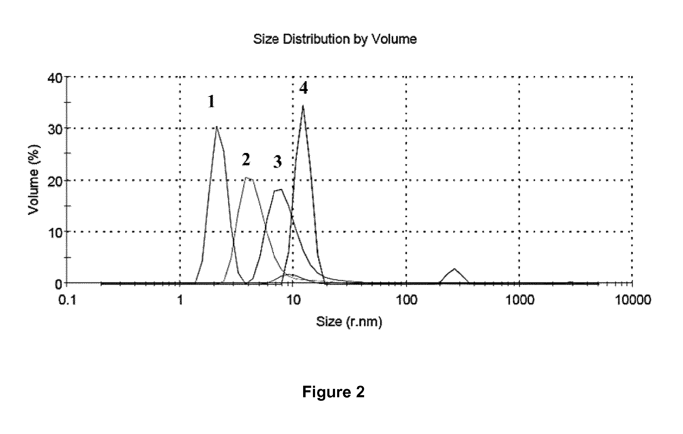

Figure 2. DLS particle size formation. The size of the particles of polymers **1-4** formed in solution was measured by dynamic light scattering (DLS). The particle size was expressed as hydrodynamic radius (Rh) in nm. The polymers were dissolved in PBS (filtered by 0.02 microns) at 25°C, 1 mg/ml. The measurements were carried out in a Malvern Zetasizer Nano-S (Zetasizer Software v6.20) and expressed as % volume per size (r. nm). The results showed an increase of the size due to aggregation of the polymers when triiodobenzene content is higher. HSA protein was used as control.

### Detailed Description of the Invention

### Compounds of the Invention

The term "biodegradable" refers to polymers that are degraded in a biological environment by either a biologically assisted mechanism or by a chemical mechanism which can occur in a biological medium. Examples of biodegradation processes include hydrolysis, enzymatic action, oxidation and reduction.

The term "biocompatible" as used herein is intended to describe compounds that exert minimal destructive or host response effects while in contact with body fluids or living cells or tissues.

In the context of the invention, the term "C₁-C₆ alkyl" refers to a saturated branched or linear alkyl chain which contains from 1 to 6, preferably form 1 to 3 (C₁-C₃ alkyl), carbon atoms and which is bound to the rest of the molecule through a single bond. Illustrative examples include methyl, ethyl, n-propyl, isopropyl, n-butyl, isobutyl, sec-butyl, tert-butyl, pentyl, hexyl, etc. Preferably, C₁-C₆ alkyl and C₁-C₃ alkyl are selected from methyl, ethyl and propyl groups.

The compounds of the invention can be in free form or in solvate form (for example, hydrates, alcoholates, etc.), both forms being included within the scope of the present invention. Solvation methods are generally well known in the state of the art. The invention also provides "salts" of the compounds described in the present description.

The term "pharmaceutically acceptable" relates to molecular entities and compositions being physiologically tolerable and normally not causing an allergic reaction or similar adverse reaction, such as gastric discomfort, dizziness and the like, when they are administered to a human being. Preferably, as used in this description, the term "pharmaceutically acceptable" means approved by a governmental regulatory agency or listed in the US pharmacopoeia or another generally recognized pharmacopoeia for use in animals, and more particularly in humans. For those persons skilled in the art, it will be evident that the scope of the present invention also includes salts which are not pharmaceutically acceptable as possible means for obtaining pharmaceutically acceptable salts.

The term "molecular weight" as used herein refers to the weight average molecular weight. It can be determined by SEC-MALS RI (size exclusion chromatography-multi angles laser scattering-refractive index).

The term "triiodobenzene content" refers to the percentage of triiodo-substituted benzene by weight with respect to the total weight of the polymer. Triiodobenzene content can be measured by ¹H-NMR analysis.

As mentioned above, an aspect of the present invention relates to a polymer having a molecular weight from 5,000 to 500,000 g/mol, which comprises randomly repeating structural units of formula (I), being each of these repeating structural units equal or different 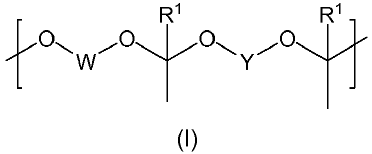 wherein R¹ is independently selected from hydrogen and C₁-C₆ alkyl; W is selected from a biradical of formula (II) 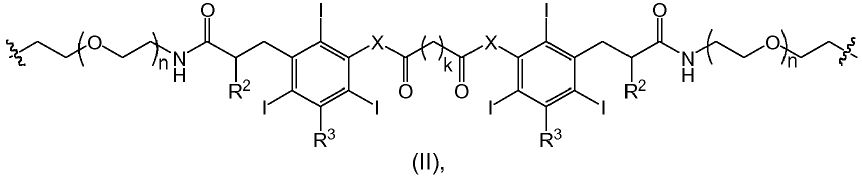 a biradical of formula (III) 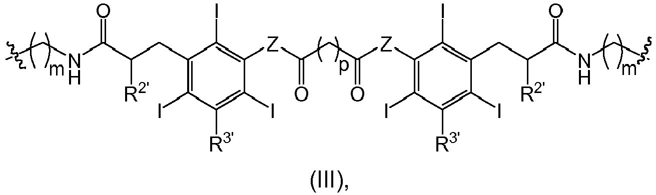 and a biradical of formula (IV)

-(CH₂CH₂O)_{q}-CH₂CH₂- (IV),

wherein
R² and R^{2'} are independently selected from hydrogen and C₁-C₆ alkyl,
R³ and R^{3'} are independently selected from hydrogen, C₁-C₆ alkyl, F, Cl and Br, X and Z are independently selected from NH and O,
n, k, m and p are independently selected from an integer from 1 to 20, and
q is an integer from 1 to 300;
Y is a biradical of formula (V)

-(CH₂CH₂O)ᵣ-CH₂CH₂- (V),

wherein r is an integer from 1 to 10;
and wherein at least one of the repeating structural units of formula (I) contains a group W selected from a biradical of formula (II) and a biradical of formula (III).

Repeating structural units of formula (I) can be equal or different. Therefore, W can be different in each of the repeating units of formula (I).

In a particular embodiment, the polymers of the present invention have a molecular weight from 10,000 to 100,000 g/mol, preferably from 20,000 to 50,000 g/mol, more preferably from 20,000 to 40,000 g/mol.

Polymers of the invention contain at least a biradical of formula (II) or a biradical of formula (III). In a particular embodiment, the polymers of the present invention have a triiodobenzene content from 0.1 to 75%, preferably from 0.1 to 50%, more preferably from 0,5 to 30%, even more preferably from 1 to 20%, by weight of the weight of the polymer, and still more preferably 2 to 15% by weight of the weight of the polymer. In a particular embodiment, the polymer of the invention has a triiodobenzene content from 3 to 15% by weight of the weight of the polymer.

In a particular embodiment, R¹ is independently selected from hydrogen and C₁-C₃ alkyl. Preferably, R¹ is hydrogen.

In a particular embodiment, Y is a biradical of formula (V) wherein r is an integer from 1 to 5, preferably from 1 to 3, more preferably r is 1.

In a particular embodiment, R² and R^{2'} are independently selected from C₁-C₃ alkyl, preferably R² and R^{2'} are ethyl.

In a particular embodiment, R³ and R^{3'} are independently selected from hydrogen, C₁-C₃ alkyl, F, Cl and Br; preferably R³ and R^{3'} are hydrogen.

In a particular embodiment, X and Z are NH.

In a particular embodiment, k and p are independently selected from an integer from 1 to 10, preferably from 2 to 6, more preferably k and p are 4.

In a particular embodiment, n is an integer from 1 to 10, preferably from 1 to 5, more preferably n is 1.

In a particular embodiment, m is an integer from 1 to 10, preferably from 1 to 5.

In a particular embodiment, R² is ethyl, R³ is hydrogen, X is NH, k is 4 and n is 1 in the biradical of formula (II).

In a particular embodiment, R^{2'} is ethyl, R^{3'} is hydrogen, Z is NH, p is 4 and m is from 1 to 5 in the biradical of formula (III).

In a particular embodiment, q is an integer from 1 to 100, preferably from 1 to 70, more preferably q is from 40 to 50.

In another embodiment, W is selected from a biradical of formula (III) as defined herein and a biradical of formula (IV) as defined herein.

In another embodiment, R¹ and Y are the same in all the repeating structural units of formula (I) and W is selected from a biradical of formula (III) and a biradical of formula (IV).

In a particular embodiment, W is selected from a biradical of formula (II) and a biradical of formula (IV). In a particular embodiment, R¹ and Y are the same in all the repeating structural units of formula (I) and W is selected from a biradical of formula (II) and a biradical of formula (IV), preferably, W is selected from a biradical of formula (II) wherein R² is C₁-C₃ alkyl, R³ is hydrogen, X is NH, k is from 2 to 6 and n is an integer from 1 to 5, and a biradical of formula (IV) wherein q is from 1 to 70. More preferably, from a biradical of formula (II) wherein R² is ethyl, R³ is hydrogen, X is NH, k is 4 and n is 1, and a biradical of formula (IV) wherein q is from 40 to 50.

In a particular embodiment, R¹ is hydrogen, Y is a biradical of formula (V) wherein r is 1, W is selected from a biradical of formula (II) wherein R² is ethyl, R³ is hydrogen, X is NH, k is 4 and n is 1, and a biradical of formula (IV) wherein q is from 40 to 50.

In a particular embodiment, the polymer of the invention consists of randomly alternating repeating structural units of formula (Ia) and (Ib):

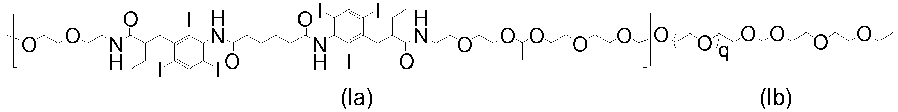

Preferably, q is 40-50.

In a particular embodiment, the polymer of the invention has a molecular weight of 20,000-40,000 g/mol and consists of randomly alternating repeating structural units of formula (Ia) and (Ib), wherein q is 40-50.

In a particular embodiment, the polymer of the invention has a molecular weight of 20,000-40,000 g/mol, a triiodobenzene content from 2 to 15% by weight of the weight of the polymer, and consists of randomly alternating repeating structural units of formula (Ia) and (Ib), wherein q is 40-50.

In another aspect, the invention is directed to a compound of formula (VI) 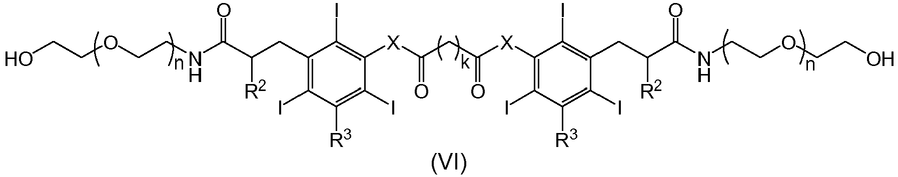 wherein R², R³, X, n and k are as defined herein, or a pharmaceutically acceptable salt or solvate thereof.

In a preferred embodiment, R² is ethyl, R³ is hydrogen, X is NH, k is 4 and n is 1.

In another aspect, the invention is directed to a compound of formula (VII) 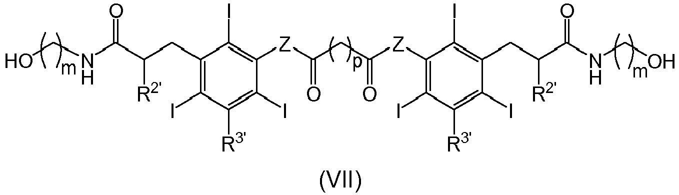 wherein R^{2'}, R^{3'}, Z, m and p are as defined herein, or a pharmaceutically acceptable salt or solvate thereof.

In a preferred embodiment, R^{2'} is ethyl, R^{3'} is hydrogen, Z is NH, p is 4 and m is from 1 to 5.

### Process for preparing the compounds of the Invention

In another aspect, the invention is directed to a process for preparing a polymer of the invention, comprising reacting one or more diols of formula (VI) and/or one or more diols of formula (VII) and/or one or more diols of formula (VIII)

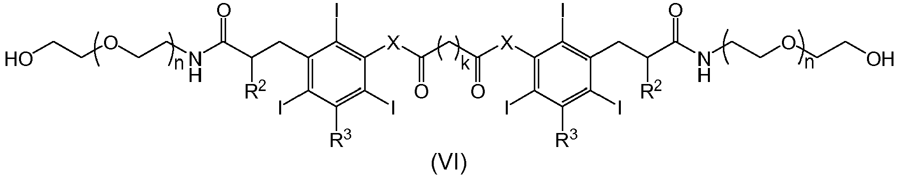

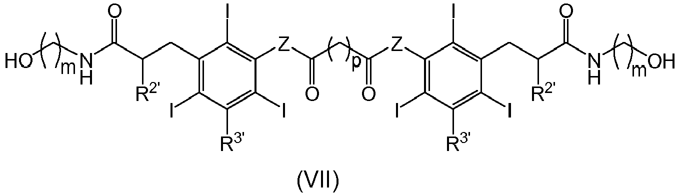

HO-(CH₂CH₂O)_{q}-CH₂CH₂-OH (VIII)

or a salt or solvate thereof,
with a compound of formula (IX) 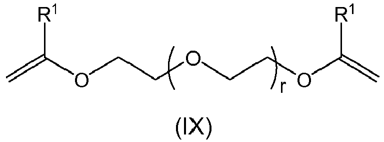 wherein R¹, R², R^{2'}, R³, R^{3'}, X, Z, n, m, k, p and r are as defined herein.

Diols of formula (VI) and (VII) can be obtained by amidation of the corresponding dicarboxylic acid derivatives using conventional methods known in the art.

The diol of formula (VIII) is preferably a polyethylene glycol (PEG) compound having a molecular weight in the range 100-10,000, preferably 200-5,000, more preferably 1,000-3,000 Da. In a particular embodiment, it is a polyethylene glycol compound having a molecular weight of 2,000 Da.

The divinyl ether of formula (IX) may be obtained commercially or may be made by conventional methods known by the skilled in the art.

The reaction can be carried out in the presence of a catalyst, preferably an acid catalyst, such as p-toluenesulfonic acid, hydrochloric acid, sulfuric acid, phosphoric acid, methanesulfonic acid, acetic acid or trifluoroacetic acid. Preferably, the reaction is carried out in the presence of p-toluenesulfonic acid.

The reaction can be performed in the presence of an organic solvent, such as a cyclic ether (e.g. 1,4-dioxane, tetrahydrofuran, methyltetrahydrofuran), a halogenated solvent (e.g. dichloromethane, chloroform), an aromatic solvent (e.g. toluene), an amide (e.g. DMF), a sulfoxide (e.g. DMSO), and mixtures thereof. In a particular embodiment, the solvent is DMF. Preferably, the solvent is anhydrous.

The reaction can be performed at a temperature between 0-200 °C, preferably between 20-100 °C, more preferably between 20 and 60 °C. In a particular embodiment, the reaction is performed at room temperature.

In a particular embodiment, the reaction is carried out in the presence of p-toluenesulfonic acid and DMF.

In another aspect, the invention is directed to a polymer obtainable by a process as defined herein.

### Pharmaceutical compositions

In another aspect, the invention is directed to a composition comprising a polymer of the invention as defined herein and a pharmaceutically acceptable carrier.

The term "pharmaceutically acceptable carrier" in the compositions according to the present invention refers to pharmaceutically acceptable solvents, suspending agents or vehicles for delivering a compound or polymer of the present invention and which are acceptable from a toxicological viewpoint. The number and the nature of the pharmaceutically acceptable carriers depend on the desired administration form. Suitable pharmaceutical carriers are known by the person skilled in the art, and they and the methods of formulating the compositions can be found in standard references (e.g. "Remington: The Science and Practice of Pharmacy", 20th edition (2003) Genaro A.R., ed., Lippincott Williams & Wilkins, Philadelphia, US).

The composition according to the present invention is suitable for X-ray contrast. It can be administered in diagnostically effective amounts by any route used for contrast product. Preferably, the composition is administered by parenteral route. In a particular embodiment, the composition is administered by intravenous injection.

In a particular embodiment, the composition is an intravenous injectable composition comprising a polymer of the invention as defined herein and a pharmaceutically acceptable liquid carrier. Pharmaceutically acceptable liquid carriers include, but are not limited to, water, saline solutions, aqueous dextrose and glycols.

The term "diagnostically effective amount" as used herein refers to an amount of the compound or contrast agent that is sufficient for its detection following administration to a subject using imaging equipment. Said amount can be determined by the health professional depending on the administered agent and the target region.

### Uses of the compounds of the Invention

The polymers of the present invention can be monitored in vivo by suitable diagnostic procedures, for example by X-ray imaging.

Therefore, another aspect of the invention refers to a polymer of the invention as defined herein for use in medicine, preferably in diagnosis.

Another aspect refers to a polymer of the invention as defined herein for use as a contrast agent, preferably in X-ray imaging.

The polymers of the invention as defined herein can be used as contrast agent for the diagnosis of cancer, preferably for the diagnosis of cancer by X-ray imaging.

The invention also refers to the use of a polymer of the invention as defined herein for use in medicine, preferably in diagnosis.

The invention also relates to the use of a polymer of the invention as defined herein for use in the preparation of a contrast agent, preferably in X-ray imaging.

In a particular embodiment, the invention refers to the use of a polymer of the invention as defined herein for use in the preparation of a contrast agent for the diagnosis of cancer, preferably for the diagnosis of cancer by X-ray imaging.

The invention also refers to a method of imaging comprising administering a polymer of the invention as defined herein to a patient, e.g. an animal or human, and detecting said polymer. The polymers of the invention can be detected using suitable imaging equipment and by methods known to those skilled in the art.

In a particular embodiment, the invention relates to a method for the diagnosis of cancer comprising administering a polymer of the invention as defined herein to a patient, e.g. an animal or human, and detecting said polymer or compound.

The polymers of the invention may be used both in *in vivo* and in *in vitro* diagnostic methods, wherein the contrast agent is used on a sample isolated from the patient. In a preferred embodiment the polymers of the invention are used for *in vivo* diagnosis.

The terms "imaging agent" and "contrast agent" are used here interchangeably and refer to a compound that increases image contrast. Its use facilitates the differentiation of different parts of the image increasing the "contrast" between those different regions of the image. Preferably, the contrast agent is an X-ray contrast agent, i.e. a compound that preferentially absorbs X-ray radiation in the target tissue and therefore improves quality of the image, thus resulting in better diagnosis of the medical condition.

The terms "cancer" and "tumor" refer to the physiological condition in mammals characterized by unregulated cell growth and include, for example, breast, heart, lung, small intestine, colon, spleen, kidney, bladder, head, neck, ovarian, prostate, brain, pancreas, skin, bone, bone marrow, blood, thymus, uterus, testicles and liver tumours. In a particular embodiment, the compounds of the invention are used in the diagnosis of solid tumors, preferably breast cancer.

The following examples illustrate the invention and must not be considered in a limiting sense thereof.

### EXAMPLES

### Example 1: Preparation of Triiodobenzene-Diol 1 derived from N,N'-adipoylbis-(3-amino-alpha-ethyl-2,4,6-triiodohydrocynnamic acid)

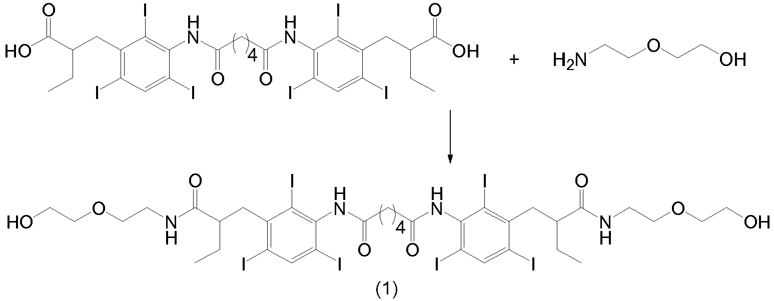

A solution of N,N'-adipoylbis-(3-amino-alpha-ethyl-2,4,6-triiodohydrocynnamic acid) (148.3 mg, 118 µmol), WSC·HCl (69 mg, 360 µmol) and HOAt (49.3 mg, 362 µmol) in DMF (0.8 ml) in an eppendorf vial was stirred at room temperature for 5 min. Then, 2-(2-aminoethoxy)-ethanol (26 µl, 261 µmol) was added. The mixture was gently stirred for 2 h at r.t. The resulting solution was added dropwise on 10 ml water to precipitate the product. The solvent was decanted and the solid was washed with additional 2x10 ml water. A white solid was obtained which was finally lyophilized. White powder, 98%. HPLC-MS analysis showed Mw 1428.1 m/z. ¹H-NMR (400 MHz, DMSO-d₆): 0.76 (6H, t), 1.34 (2H, m), 1.66 (2H, m), 1.76 (4H, m), 2.34 (4H, m), 2.42 (2H, m), 3.16 (4H, m), 3.22 (4H, m), 3.39 (8H, m), 3.47 (4H, m), 4.55 (2H, m), 7.81 (2H, m), 8.35 (2H, s), 9.86 (2H, d).

### Example 2: Preparation of the polymer 2, having randomly alternating repeating structural units of formula (Ia) and (Ib)

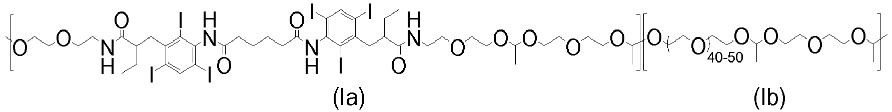

A solution of pTSA (dried overnight under vacuum at 35 °C, 17 mg, 0.089 mmol), compound **1** (75.3 mg, 0.053 mmol) and PEG 2 kDa (0.799 g, 0.400 mmol) in 1 ml DMF (anhydride) was stirred for 5 min. at room temperature, in the presence of molecular sieve (4 Armstrong), in a 10 mL round-bottom flask (dried overnight at 110 °C), which was sealed with a septum and purged with Argon. Then, diethylene glycol divinyl ether (66 µL, 0.404 mmol) was added and the mixture was stirred slowly in the dark. Up to an additional equivalent of diethylene glycol divinyl ether (66 µL, 0.404 mmol) was added after 1 h of reaction, and another equivalent (66 µL, 0.404 mmol) after 2 h of reaction. A viscous solution was obtained after 5 h. The reaction was quenched with Et₃N (290 µL) and stirred for 10 min at room temperature. The viscous solution was precipitated in Et₂O (50 mL) and washed again 2x50 mL with additional Et₂O until a white solid was obtained (900 mg). The solid obtained was dissolved in 50 mM (NH₄)₂CO₃ (300 mL) and filtered through a controlled porous membrane (MWCO 5 kDa) in an Amicon 8400 (Millipore®) system by application of Argon pressure (2-3 bar). The solution was concentrated to 75 mL and purification/filtration process was carried out three times more. The final concentrate was lyophilized. The molecular weight was Mw = 34.5 kDa and Mw/Mn (PDI) = 1.38 as determined by SEC-MALS RI (dn/dc = 0.135). ¹H-NMR (400MHz, DMSO-d₆) for the acetal formed: 1.17 (3H, d, acetal CH₃) and 4.68 (1H, q, acetal CH). The Triiodobenzene content was quantified by ¹H-NMR (CH₃ signal at 0.78, t): 3 % w/w triiodobenzene.

Polymers **3** and **4** having the same structural components but varying the triiodobenze (TIB) content, as well as comparative polymer **1** lacking trioodobenzene, were obtained by a similar process as the one described above for polymer **2** but using the following amounts:

| **Polymer** | **pTSA (mg) [mmol]** | **PEG 2KDa (mg) [mmol]** | **Diethylenglycoldivinyl ether (µl) [mmol]** | **Compound 1 (mg) [mmol]** | **DMF (ml)** |
|---|---|---|---|---|---|
| Polymer **1** | 9.9 [0.052] | 1000 [0.5] | 123 [0.753] | 0 | 1.4 |
| Polymer **3** | 21 [0.111] | 805 [0.403] | 213 [1.303] | 152.8 [0.107] | 1 |
| Polymer **4** | 25.2 [0.133] | 799.6 [0.4] | 220 [1.346] | 296.1 [0.208] | 1 |

### Example 3: Particle formation

Particles of polymers **2-4** were formed in solution. The polymers were dissolved in PBS (solvent filtered by 0.02 microns) at 25°C, 1 mg/ml. The size of the particles was measured by dynamic light scattering (DLS). The particle size was expressed as hydrodynamic radius (Rh) in nm. The measurements were carried out in a Malvern Zetasizer Nano-S (Zetasizer Software v6.20) and expressed as % volume per size (r. nm). HSA protein was used as control.

| **Sample** | **TIB content (w/w)** | **Mw (KDa) [PDI]** | **Size (Rh, nm)** | **±SD** | **Population (%)** |
|---|---|---|---|---|---|
| HSA | - | 66 [1.0] | 3.57 | - | 100 |
| Polymer **1** | 0.0% | 62 [1.38] | 2.20 | 0.93 | 92.9 |
| Polymer **2** | 3.0% | 34 [1.63] | 4.84 | 0.27 | 99.5 |
| Polymer **3** | 6.5% | 28 [2.20] | 9.22 | 3.14 | 97.8 |
| Polymer **4** | 11.4% | 28 [1.24] | 11.79 | 0.41 | 94.9 |

The results obtained showed that the presence of the triiodobenzene moiety results in the formation of polymer particles having a particle size not lower than 3.57 nm which helps minimize renal elimination of the copolymer. It is also observed that the particle size of the particles formed by aggregation increases when the triiodobenzene content also increases.

## Claims

1. A polymer having a molecular weight from 5,000 to 500,000 g/mol which comprises repeating structural units of formula (I), being each of these repeating structural units equal or different 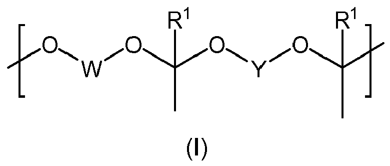 wherein
R¹ is independently selected from hydrogen and C₁-C₆ alkyl; W is selected from a biradical of formula (II) 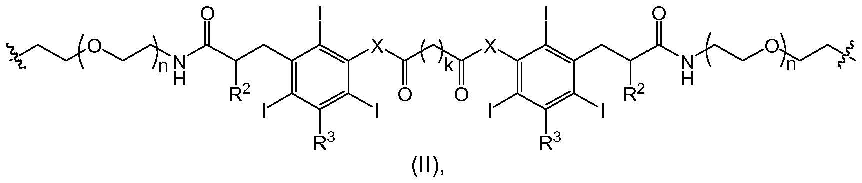 a biradical of formula (III) 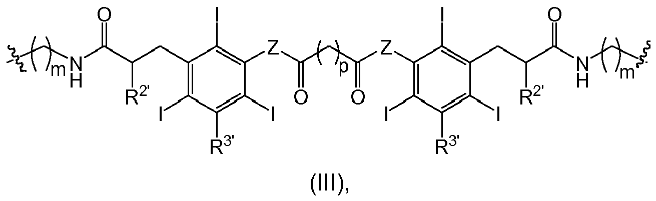 and a biradical of formula (IV)
-(CH₂CH₂O)_{q}-CH₂CH₂- (IV),
wherein
R² and R^{2'} are independently selected from hydrogen and C₁-C₆ alkyl,
R³ and R^{3'} are independently selected from hydrogen, C₁-C₆ alkyl, F, Cl and Br, X and Z are independently selected from NH and O, n, k, m and p are independently selected from an integer from 1 to 20, and
q is an integer from 1 to 300;
Y is a biradical of formula (V)
-(CH₂CH₂O)ᵣ-CH₂CH₂- (V),
wherein r is an integer from 1 to 10; and wherein at least one of the repeating structural units of formula (I) contains a group W selected from a biradical of formula (II) and a biradical of formula (III).

2. Polymer according to claim 1, wherein R¹ is hydrogen.

3. Polymer according to any one of the previous claims, wherein r is an integer from 1 to 3.

4. Polymer according to any one of the previous claims, wherein R² and R^{2'} are independently selected from C₁-C₃ alkyl.

5. Polymer according to any one of the previous claims, wherein R³ and R^{3'} are hydrogen.

6. Polymer according to any one of the previous claims, wherein
R¹ is hydrogen,
Y is a biradical of formula (V) wherein r is 1,
W is selected from a biradical of formula (II) wherein R² is ethyl, R³ is hydrogen, X is NH, k is 4 and n is 1, and a biradical of formula (IV) wherein q is 40-50.

7. Polymer according to any one of the previous claims, which consists of repeating structural units of formula (Ia) and (Ib) 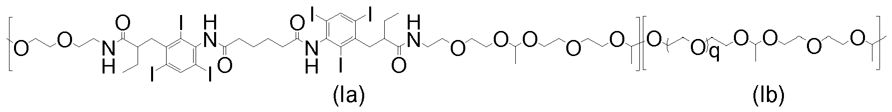 wherein q is 40-50.

8. Polymer according to any one of the previous claims, having a molecular weight of from 10,000 to 100,000 g/mol.

9. Polymer according to any one of the previous claims, wherein the total triiodobenzene content is from 0.1 to 75%.

10. A compound selected from a compound of formula (VI) and a compound of formula (VII) 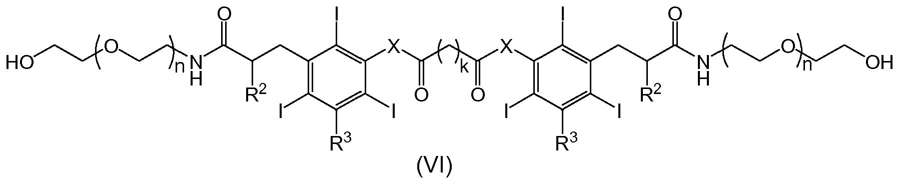 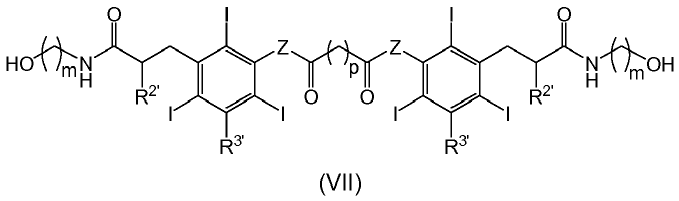 wherein R², R^{2'}, R³, R^{3'}, X, Z, n, k, m and p are as defined in claim 1, or a pharmaceutically acceptable salt or solvate thereof.

11. A process for preparing a polymer as defined in any of claims 1 to 9, comprising reacting a diol of formula (VI) and/or a diol of formula (VII) as defined in claim 10 and/or a diol of formula (VIII)
HO-(CH₂CH₂O)_{q}-CH₂CH₂-OH (VIII)
or a salt or solvate thereof,
with a compound of formula (IX) 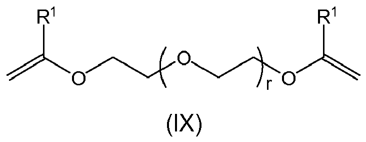 wherein R¹, q and r are as defined in claim 1.

12. A composition comprising a polymer as defined in any of claims 1 to 9 and a pharmaceutically acceptable carrier.

13. A polymer as defined in claims 1 to 9, for use in medicine.

14. A polymer as defined in claims 1 to 9, for use as a contrast agent.

15. A polymer according to claim 14, for use as a contrast agent for the diagnosis of cancer.
